# EUROPÄISCHE PATENTANMELDUNG

(11) **EP 3 409 339 A1**
(43) Veröffentlichungstag der Anmeldung: **05.12.2018**
(21) Anmeldenummer: 17173304.1
(22) Anmeldetag: 29.05.2017
(51) Int. Cl.: B01D 15/18

(54) **VERFAHREN ZUM TRENNEN VON NATURSTOFFGEMISCHEN MITTELS SCPC**

(71) Anmelder: Bionorica Ethics GmbH, 92318 Neumarkt (DE)
(72) Erfinder: Englert, Michael, 90478 Nürnberg (DE); Rutz, Andreas, 91741 Dornhausen (DE)
(74) Vertreter: Simandi, Claus

(57) **Zusammenfassung**

Die Erfindung betrifft ein Verfahren zum Trennen von Naturstoffgemischen, insbesondere solche aus Pflanzenextrakten, und deren Isolierung und Aufreinigung als auch Gewinnung mittels Sequential Centrifugal Partition Chromatography (sCPC).

## Beschreibung

Die Erfindung betrifft ein Verfahren zum Trennen von Naturstoffgemischen, insbesondere solche aus Pflanzenextrakten und deren Isolierung und Aufreinigung als auch Gewinnung mittels Sequential Centrifugal Partition Chromatography (sCPC).

Der kontinuierliche TMB (True Moving Bed) Prozess als Gegenstromverfahren verbindet die Vorteile der Flüssig-Flüssig Chromatographie mit denen eines kontinuierlichen Prozesses, wie zum Beispiel dem SMB (Simulated Moving Bed) unter Einsatz einer Zentrifugalsäule bzw. rotierende Trennsäule. Bei der Flüssig-Flüssig Chromatographie wird sowohl mit flüssiger mobiler Phase, als auch mit flüssiger stationärer Phase gearbeitet und kein festes Säulenmaterial eingesetzt. Im Gegensatz zur SMB und deren verwandten Prozessen wird bei einer TMB die Bewegung der nicht-mischbaren Phasen nicht über eine Ventilschaltung erreicht, sondern über eine getaktete Phasenumkehr in einer ausgewählten Frequenz. Die grundlegende technische Lehre ist in WO 2005/011835 A1 offenbart.

Charakteristisch ist der kontinuierliche Wechsel der stationären Phase zur mobilen Phase und umgekehrt, d.h. es kann die dichtere oder weniger dichte Phase als mobile Phase ausgewählt werden und ein Wechsel dieser Auswahl ist während einer Trennung möglich. Daher wird diese präparative Chromatographie als "Sequential Centrifugal Partition Chromatography (sCPC)" bezeichnet. Die zu trennenden Stoffe A und B werden in einer Flüssig-Flüssig-Zentrifugalsäule aufgrund eines unterschiedlichen Verteilungskoeffizienten mit verschiedenen Geschwindigkeiten in den beiden Phasen verschiedener Dichte bewegt.

Eine sCPC-Apparatur weist mindestens einen Rotor mit vielen runden Metallplatten auf in denen sich beispielsweise über Eintausend in Serie verbundene Trennkammern (auch Rotorkammern genannt unter Ausbildung einer rotierenden Trennsäule) befinden. Mit Hilfe einer Pumpe wird im Rotor die stationäre flüssige Phase von einer mobilen flüssigen Phase (kontinuierlich) durchströmt. Der Rotor wird z.B. auf ca. 1.000 U/min und mehr beschleunigt, so dass die Zentrifugalkraft die Trennung der beiden flüssigen Phasen aufgrund der Dichteunterschiede in den einzelnen Kammern bewirkt. Sobald die flüssige mobile Phase im Gleichgewicht (Equilibrium) mit der flüssigen stationären Phase liegt, kann die Probe bzw. das Substanzgemisch in den Rotor injiziert werden.

Die Stofftrennung erfolgt aufgrund verschiedener Adsorption in der mobilen bzw. stationäre Phase oder des jeweiligen Verteilungskoeffizienten (K) eines Stoffes zur mobilen/stationären Phase, wobei die Stoffe durch die einzelnen Trennkammern zum Rotorausgang bewegt und fraktioniert werden. Der charakteristische alternierende Wechsel von stationärer Phase und mobiler Phase erfolgt durch Steuerung der eingesetzten Pumpen mittels Ventilen in einer zu wählenden Frequenz. In einer zu wählenden Zeitdauer ("Duration of pumping, Pumpdauer") wird zu der jeweils angesteuerten Phase das zugehörige Lösungsmittel eingepumpt und zwar jeweils an den beiden Enden eines Rotors, so dass die Pumpen entgegengesetzt angeordnet sind.

Die Probe bzw. das Substanzgemisch wird vorzugsweise intermediär in den Rotor eingespeist. In einer weiteren Ausführungsform können zwei oder mehrere Rotoren gekoppelt werden.

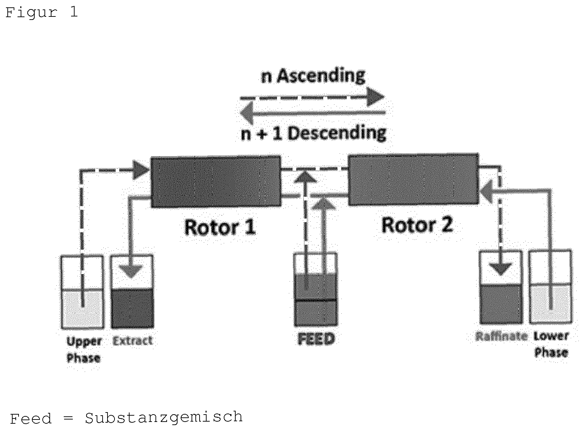

Ein prinzipieller Zyklus stellt sich in Figur 1 abgebildet dar.

Die Rotorkammern werden vorzugsweise im Verhältnis 50/50 (Volumen %) mit einer oberen und einer unteren flüssigen Phase befüllt. Das Substanzgemisch wird mittels Pumpen kontinuierlich zwischen die beiden rotierenden Trennsäulen mit einer definierten Flussrate eingespeist und die Trennung erfolgt zyklisch und zeitversetzt, wobei in einem ersten Schritt die obere Phase als mobile Phase dient ("Ascending") und in einem zweiten Schritt die untere Phase als mobile Phase dient ("Descending").

Entsprechend der Konzentration und der unterschiedlichen Verteilungskoeffizienten der Substanzen im Substanzgemisch erfolgt die Auftrennung in zwei Produktströme, siehe Figur 1.

Geeignete Geräte und Apparaturen können von Armen Technologies (Frankreich) unter dem Namen "True Moving Bed CPC" erhalten werden.

Im Stand der Technik sind zu sCPC die Aufreinigung von Modellsubstanzen und binären Gemischen beschrieben (Goll, Johannes, Andreas Frey, and Mirjana Minceva. "Study of the separation limits of continuous solid support free liquidliquid chromatography: Separation of capsaicin and dihydrocapsaicin by centrifugal partition chromatography." Journal of Chromatography A 1284 (2013): 59-68; Hopmann E, Goll J, Minceva M. Sequential centrifugal partition chromatography: A new continuous Chromatographie technology. Chem Eng Technol. 2012;35(1):72-82), jedoch nicht Verfahren zur Trennung und/oder Reinigung von Naturstoffen aus Pflanzenextrakten und zwar zur Herstellung von Fraktionen und Reinstoffen.

Daher betrifft die Erfindung ein Verfahren zur Trennung und/oder Reinigung von Naturstoffen aus Pflanzenextrakten, enthaltend mindestens einen Flüssig-flüssig-Verteilungschromatographieschritt, wobei ein kontinuierlicher Wechsel der stationären Phase zur mobilen Phase und umgekehrt erfolgt (sCPC), wobei eine oder mehrere Fraktionen abgetrennt wird/werden.

Daher betrifft die Erfindung ein Verfahren zur Trennung und/oder Reinigung von Naturstoffen aus Pflanzenextrakten, enthaltend mindestens einen Flüssig-flüssig-Verteilungschromatographieschritt, wobei ein kontinuierlicher Wechsel der stationären Phase zur mobilen Phase und umgekehrt erfolgt (sCPC), wobei mindestens ein Reinstoff abgetrennt wird.

Nachstehende Gattungen sind für die Pflanzenextrakte erfindungsgemäß bevorzugt:
*Equiseti, Juglandis, Millefolii, Quercus, Taraxaci, Althaeae, Matricariae, Centaurium, Levisticum, Rosmarinus, Angelica, Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis, Rumicis, Verbena,Sambucus, Gentiana, Cannabis, Silybum.*

Nachstehende Arten sind für die Pflanzenextrakte erfindungsgemäß bevorzugt:
*Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamilleblüten)), *Centaurium erythraea (Tausendgüldenkraut), Levisticum officinale (Liebstöckel), Rosmarinus officinalis (Rosmarin), Angelica dahurica (Sibirische Engelwurz, PinYin Name: Baizhi), Angelica sinensis (Chinesische Angelika, PinYin Name: Danggui), Artemisia scoparia (Besen-Beifuß, PinYin Name: Yinchen), Astragalus membranaceus (var. Mongolicus) (Traganthwurzel, Chin.: Huang-Qi), Leonurus japonicus (Löwenohr, Chin.: T'uei), Salvia miltiorrhiza (Rotwurzel-Salbei, Chin.: Danshen), Saposhnikovia divaricata (Siler, PinYin Name: Fangfeng), Scutellaria baicalensis (Baikal-Helmkraut, Banzhilian), Siegesbeckia pubescens (Himmlisches Kraut, PinYin Name: Xixianciao), Armoracia rusticana (Meerettich), Capsicum sp. (Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfrüchte, Chin.: Chuan Lian Zi), Olea europaea (Olive), Pelargonium sp. (Pelagornien), Phytolacca americana (Kermesbeeren), Primula veris (Schlüsselblume), Salix sp. (Weide), Thymus L. (Thymian), Vitex agnus castus (Mönchspfeffer), Vitis vinifera (Edle Weinrebe),* Rumicis herba (Sorrel herb), *Verbena officinalis* (Eisenkraut), *Sambucus nigra* (Schwarzer Holunder), *Gentiana lutea* (Gelber Enzian), *Cannabis sativa* (Hanf), *Silybum marianum* (Mariendistel).

Ebenfalls sind Mischungen der vorstehenden Gattungen und / oder Arten erfindungsgemäß umfasst.

Vorstehende Arten und Gattungen sind besonders reich an heilbringenden Naturstoffen und sind als Arzneimittelpflanzen beschrieben, wie z.B. in den Produkten auf der Basis von Pflanzenextrakten der Bionorica SE (z.B. Bronchipret®, Imupret®, Sinupret®). Ferner weisen solche Pflanzen übliche charakteristische Stoffklassen auf, wie Flavonoide, Polyphenole, etc.

In einer bevorzugten Ausführungsform wird der Pflanzenextrakt aus einem ersten Lösungsmittel, wie Alkohole, Ethanol, Wasser, Kohlenwasserstoffe, Heptan oder Mischungen davon gewonnen, und die löslichen Anteile in dem erfindungsgemäßen Verfahren eingesetzt (nach-, vorstehend: Substanzgemisch).

Zum erfindungsgemäßen Verfahren zur Trennung und/oder Reinigung von Naturstoffen aus Pflanzenextrakten mittels sCPC werden die kritischen Prozessparameter, nämlich Flussrate (flow rate) der oberen Phase, Flussrate der unteren Phase, Substanzgemisch-Flussrate, Zykluszeit "Descending" und Zykluszeit "Ascending" vorzugsweise gemäß folgenden Schritte durchgeführt:
- Festlegen einer definierten Konzentration des Substanzgemisches im ausgewählten Lösungsmittelsystem;
- Festlegen der maximalen Flussrate für die untere und obere Phase bei der kein unkontrolliertes Phasenbluten der flüssigen Phasen stattfindet;
- Festlegen der maximalen Substanzgemisch-Flussrate;
- Anpassen der Zykluszeit des Descending und / oder Ascending Modus um den Schnittpunkt der Trennung im Chromatogramm beliebig zwischen unterschiedlichen Substanzen zu verschieben, vorzugweise werden kurze Schaltzeiten verwendet um ein unkontrolliertes Phasenbluten und Austreten von nicht aufgetrennten Substanzen zu vermeiden.

Dieses Vorgehen erlaubt die gezielte An- und Abreicherung als auch Abtrennung von Fraktionen oder von Reinstoffen, wie zum Beispiel nicht abschließend Cannabidiol, Tetrahydrocannabinol, Casticin, Gentiopikrosid, Agnusid, u.a.

Beispielsweise können in den Fraktionen solche Stoffklassen von Pflanzenextrakten, wie Alkaloide, Bitterstoffe, Anthocyanine, Anthrachinone, Coumarine, Flavonoide, Glucosinolate, Lactone, Lignane, Lipide, Cannabinoide, Phenole, Polyphenole, Saponine, Terpene, Xanthone an- oder abgereichert werden, die abgetrennt werden können.

Besonders vorteilhaft können solche Fraktionen und Reinstoffe in hoher Reinheit und Ausbeute erhalten werden.

Zum Einsatz kommende Lösungsmittel können bei der Flüssig-Flüssig Verteilungschromatographie beispielsweise Skalicka-Wozniak K, Garrard I, A comprehensive classification of solvent systems used for natural product purifications in countercurrent and centrifugal partition chromatography, Nat Prod Rep. 2015 Nov;32(11):1556-61 entnommen werden.

Erfindungsgemäße Beispiele für geeignete Lösungsmittel aus denen zweiphasige Lösungsmittelsysteme (mobile Phase / stationäre Phase) für Pflanzenextrakte bereitgestellt werden können, sind:
a.) Kohlenwasserstoffe wie n-Hexan, Cyclohexan, Isohexan, Heptan, Isooctan;
b.) Ether wie t-butylmethylether, Petrolether, Diethylether;
c.) Halogenierte Lösungsmittel wie Chloroform, Dichlormethan, Benzotrifluorid, Dichlorethan, Tetrachlormethan, Trichlorethan;
d.) Wasser-Lösliche Alkhole wie Butanol, Methanol, Ethanol, Isopropanol;
e.) Wasser-Lösliche Ester wie Ethylacetat, Isopropylacetat;
f.) Acetonitril, Toluol.

Aus diesen vorgenannten Lösungsmitteln lassen sich geeignete zweiphasige Lösungsmittelsysteme herstellen, vorzugsweise solche wie:
n-Heptan/Acetonitril
n-Heptan/Ethylacetat/Acetonitril;
n-Heptan/Ethylacetat/t-Butylmethylether/ Acetonitril;
n-Heptan/Ethylacetat/Methanol/Wasser;
n-Heptan/Ethanol/Wasser.

Daher betrifft die Erfindung ebenfalls ein Verfahren zur Trennung und/oder Reinigung von Naturstoffen aus Pflanzenextrakten, enthaltend mindestens einen Flüssig-flüssig-Verteilungschromatographieschritt, wobei ein kontinuierlicher Wechsel der stationären Phase zur mobilen Phase und umgekehrt erfolgt, und die stationäre Phase als auch mobile Phase aus mindestens zwei Lösungsmitteln ausgewählt sind aus der Gruppe Kohlenwasserstoffe mit 5 - 8 Kohlenstoffatomen, insbesondere n-Heptan, Acetonitril, Ethylacetat, t-Butylmethylether, Alkohole, insbesondere Methanol, Wasser, Ethanol, so dass ein zwei-Phasen-System erhalten werden kann.

Nachfolgende Beispiele und Figuren dienen ausschließlich zur Erläuterung der Erfindung, ohne die Erfindung auf diese Beispiele zu beschränken.

### Beispiel 1:

### Darstellung eines Equipments:

### Ausrüstung:

Kontinuierlicher Flüssig-Flüssig Chromatograph True-Moving-Bed (TMB) 500 System, mit 2x250 mL Rotor, Drehzahl 100-3000 rpm, bis 100 mL/min Flussrate, 100 bar Maximaldruck von Armen Instruments (Saint-Ave, Frankreich) oder 12 L Produktionsanlage mit 2x6L Rotor.

### Zusatzausrüstung:

2xEluenten-Pumpe, bis 100 mL/min Flussrate; 2xProbenaufgabe-Pumpe, bis 50 mL/min Flussrate; 2xDioden-Array-Detektor Ecom, 4 Wellenlängen, 200-600 nm Detektionsbereich, 6 kg; 2xFraktionensammler LS-5600, inkl. Rack-Set aus Edelstahl, 20 kg

### Funktionsweise:

Die Probe bzw. das Substanzgemisch wird kontinuierlich, phasenweise auf beide Rotoren injiziert und die Probenkomponenten nach Polarität und gemäß ihrer flüssig-flüssig-Verteilungskoeffizienten K mit einem geeigneten Zweiphasigen Lösungsmittelsystem getrennt. Nach Anfahren der Anlage stellt sich ein stationärer, steady-state Zustand ein. An einem Rotor kann anschließend mittels eines Fraktionensammlers Produktseite A entnommen werden. Abwechselnd kann am zweiten Rotor mit einem weiteren Fraktionensammler Produktseite B entnommen werden kann. Die Detektoren dienen zur Überwachung der Trennung.

### Beispiel 2:

### Nutzhanfextrakt unter Abreicherung von THC

Gewinnung von Nutzhanfextrakt THC-befreit: Extraktion Cannabis herbe mittels CO2, Decarboxylierung bei 120°C im Vakuum, Auflösen in n-Heptan/Ethanol/Wasser,

| | |
|---|---|
| Methode: | |
| Parameter: | |
| Rotation: | 2600 rpm |
| Ascending Modus: | |
| Flow rate Elution: | 30 ml/min |
| Flow rate Injection: | 10 ml/min / 5 ml/min |
| Pumpdauer: | 90 sec |
| Descending Modus: | |
| Flow rate Elution: | 30 ml/min |
| Flow rate Injection: | 10 ml/min / 5 ml/min |
| Pumpdauer: | 99 sec |

| | |
|---|---|
| Flow rate = Flussrate | |

### HPLC-Chromatogramm Ausgangsmaterial:

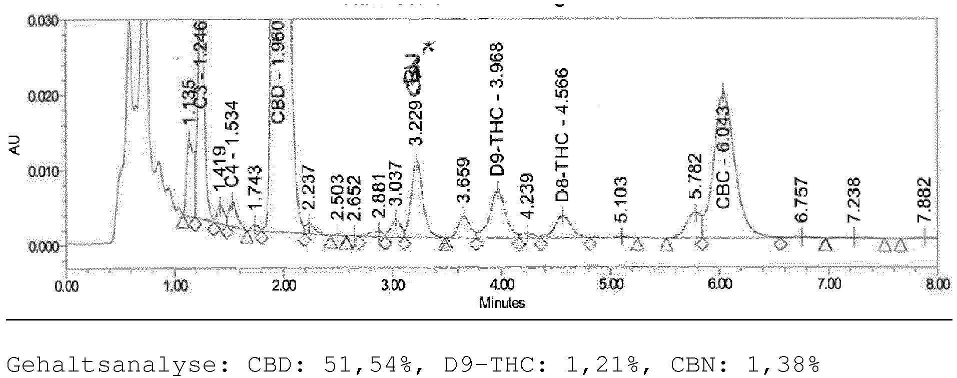

### Batch Verteilungschromatographie (CPC)

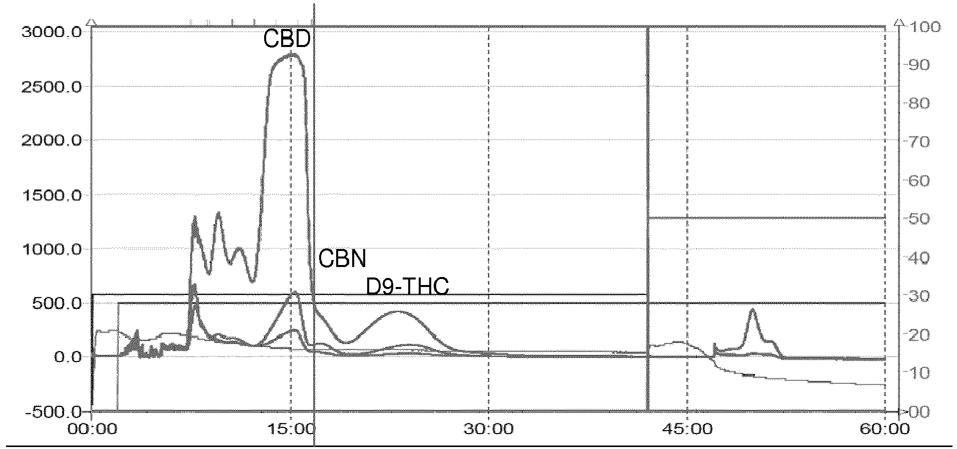

Trennung von 3,05 g Ausgangsprodukt (50g/L) in 30 min in Batchbetrieb mit n-Heptan/Ethanol/Wasser (5:4:1.4) - die vertikale Linie bei 17:00 beschreibt die Trennlinie bei der späteren kontinuierlichen Verteilungschromatographie.

### Kontinuierliche Verteilungschromatographie (sCPC):

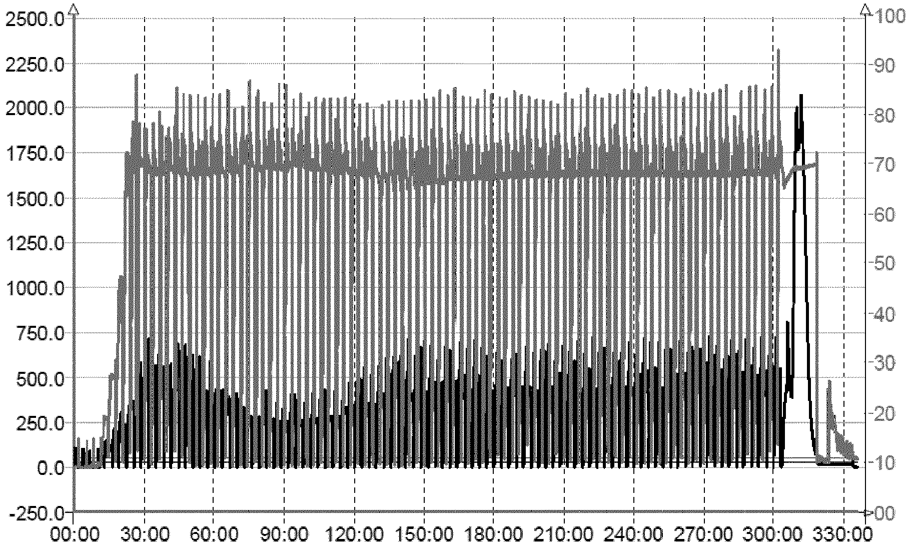

Trennung im kontinuierlichen Betrieb (50g/L) mit der SCPC mit n-Heptan/Ethanol/Wasser (5:4:1.4)
Grau: UV-Signal Descending Seite, Schwarz: UV-Signal Ascending Seite

### HPLC-Chromatogramm der Ascending Seite

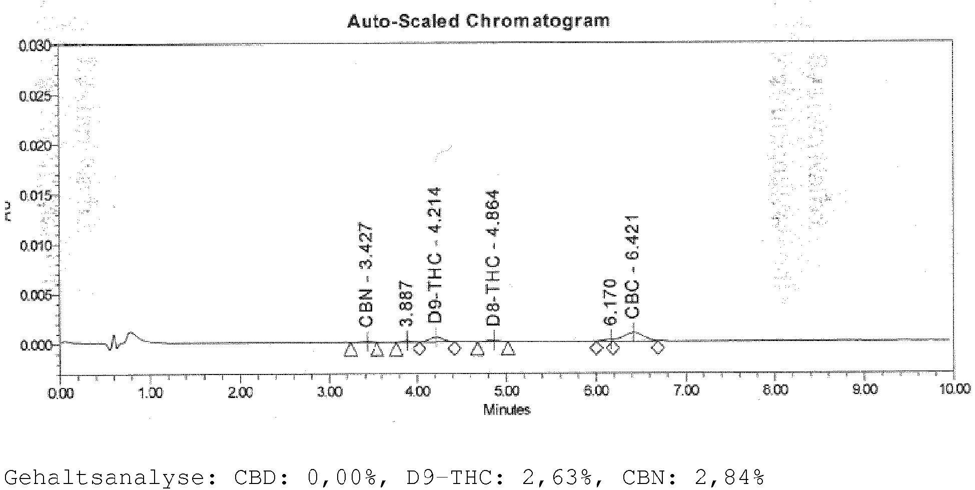

### HPLC-Chromatogramm der Descending Seite

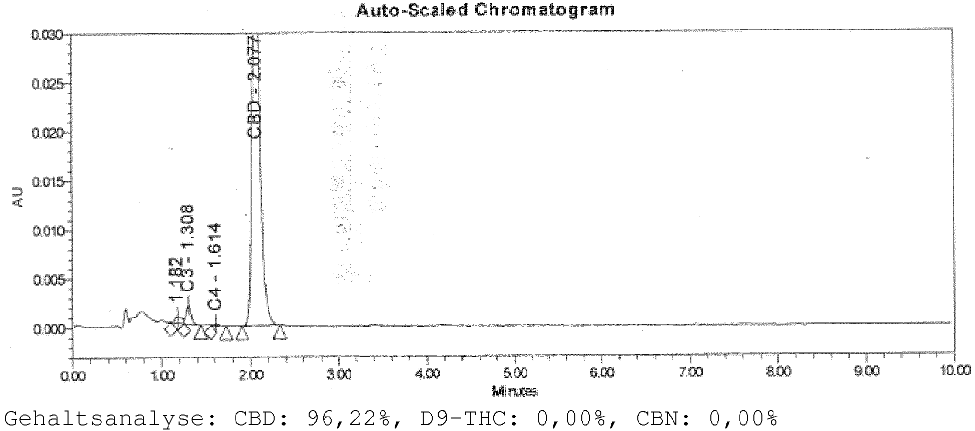

### Vergleich der Produktivitäten bei Nutzhanfextrakt Trennung:

| Anlage | Massenstrom [g/h] | Lösungsmittelverbrauch [L/h] | Verbrauch pro g Produkt [L/g] |
|---|---|---|---|
| Kontinuierliche True Moving Bed 2x250 mL Rotor | 34,81 | 8,1 | 0,23 |
| Batch CPC 500 mL Rotor | 6,10 | 1,8 | 0,30 |

### Beispiel 3:

### Aufreinigung von Casticin

| | |
|---|---|
| Methode: | |
| Parameter: | |
| Rotation: | 2600 rpm |
| Ascending Modus: | |
| Flow rate Elution: | 15 ml/min |
| Flow rate Injection: | 5 ml/min / 5 ml/min |
| Pumpdauer: | 30 sec |
| Descending Modus: | |
| Flow rate Elution: | 15 ml/min |
| Flow rate Injection: | 5 ml/min / 5 ml/min |
| Pumpdauer: | 60 sec |

### HPLC Chromatogramm Ausgangsprodukt Essi Agni Casti

Lösungsmittelsystem: n-Heptan / Ethanol / Wasser (5 : 4 : 1) (v / v / v)

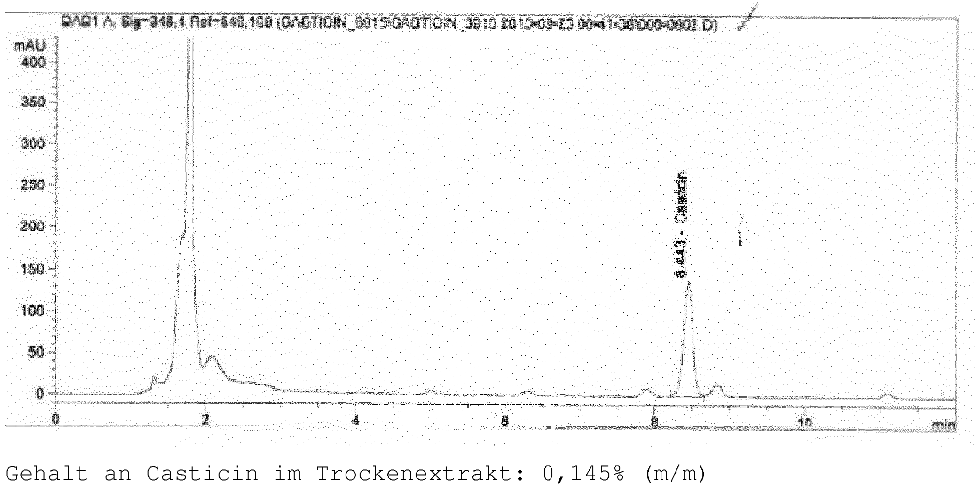

### Batch Verteilungschromatographie (CPC):

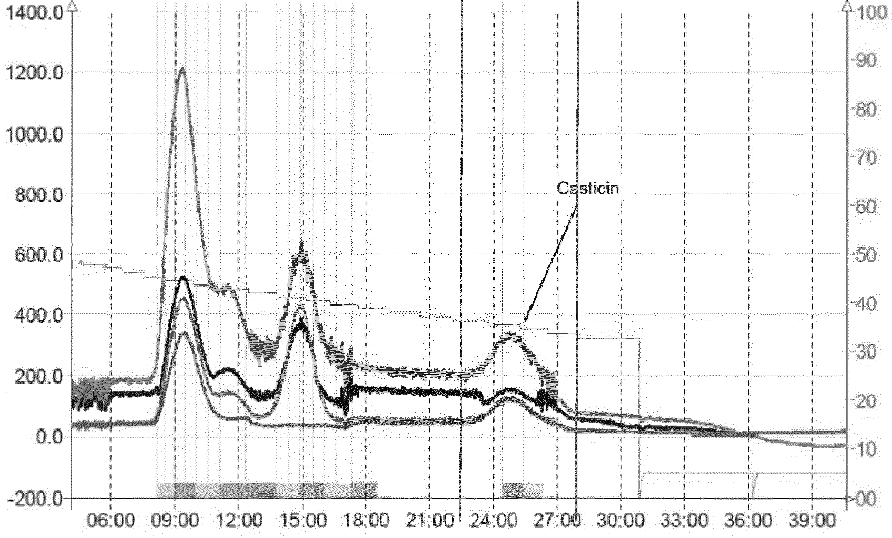

Trennung von 1 g Essi Agni Casti im Batchbetrieb mit n-Heptan/Ethylacetat/Ethanol/Wasser (7:10:7:10) - die vertikalen Linien bei 22:00 und 28:00 beschreiben die Trennungen bei der späteren kontinuierlichen Verteilungschromatographie.

### True Moving Bed Aufreinigungsschritt 1 zur Voranreicherung:

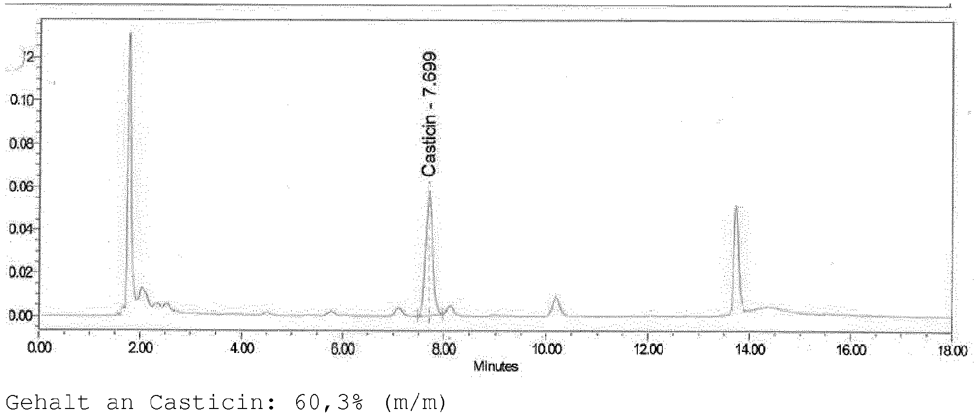

### True Moving Bed Aufreinigungsschritt 2 zur Voranreicherung:

### DAD Spektrum (oben) und HPLC Chromatogramm (unten) aufgereinigtes Casticin nach TMB

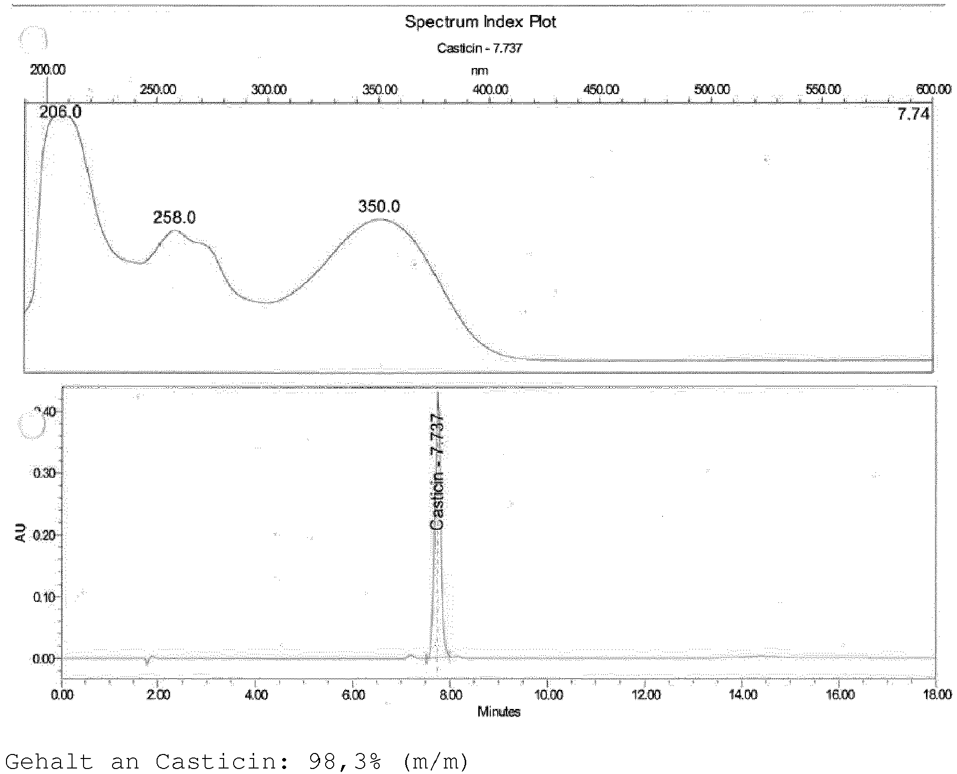

### HPLC-Chromatogramm des Ausgangsproduktes Essi Agni Casti:

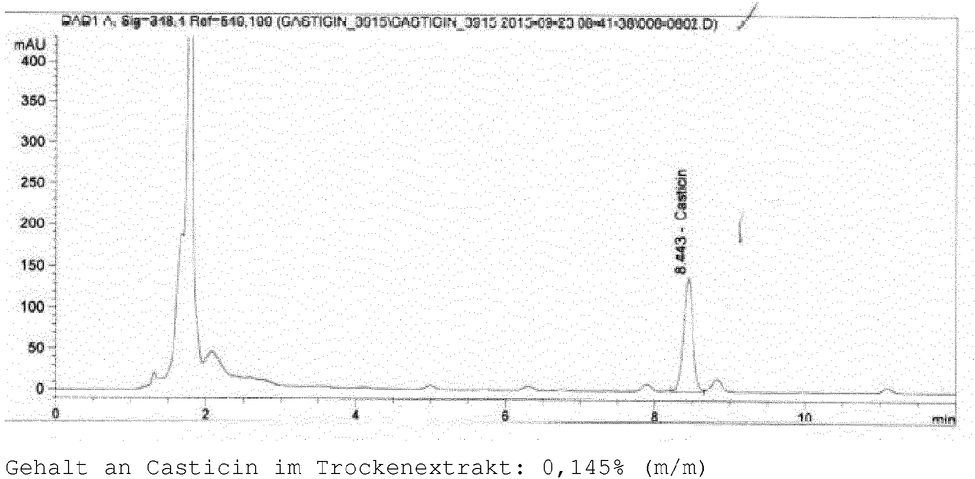

Lösungsmittelsystem: n-Heptan / Ethylacetat / Ethanol / Wasser (7 : 10 : 7: 10) (v / v / v / v)

## Patentansprüche

1. Verfahren zur Trennung und/oder Reinigung von Naturstoffen aus Pflanzenextrakten, enthaltend mindestens einen Flüssig-flüssig-Verteilungschromatographieschritt, wobei ein kontinuierlicher Wechsel der stationären Phase zur mobilen Phase und umgekehrt erfolgt, **dadurch gekennzeichnet, dass** eine oder mehrere Fraktionen abgetrennt wird/werden.

2. Verfahren zur Trennung und/oder Reinigung von Naturstoffen aus Pflanzenextrakten, enthaltend mindestens einen Flüssig-flüssig-Verteilungschromatographieschritt, wobei ein kontinuierlicher Wechsel der stationären Phase zur mobilen Phase und umgekehrt erfolgt, **dadurch gekennzeichnet, dass** mindestens ein Reinstoff abgetrennt wird.

3. Verfahren zur Trennung und/oder Reinigung von Naturstoffen aus Pflanzenextrakten nach Anspruch 1 oder 2, **dadurch gekennzeichnet, dass** mindestens ein Rotor eingesetzt wird.

4. Verfahren zur Trennung und/oder Reinigung von Naturstoffen aus Pflanzenextrakten nach einem der vorhergehenden Ansprüche, **dadurch gekennzeichnet, dass** der Pflanzenextrakt aus einem ersten Lösungsmittel, wie Alkohole, Ethanol, Wasser, Kohlenwasserstoffe, Heptan oder Mischungen davon gewonnen wird, und die löslichen Anteile eingesetzt werden.

5. Verfahren zur Trennung und/oder Reinigung von Naturstoffen aus Pflanzenextrakten nach einem der vorhergehenden Ansprüche, **dadurch gekennzeichnet, dass** der Reinstoff ausgewählt ist aus der Gruppe Cannabidiol, Tetrahydrocannabinol, Casticin, Gentiopikrosid, Agnusid.

6. Verfahren zur Trennung und/oder Reinigung von Naturstoffen aus Pflanzenextrakten nach einem der vorhergehenden Ansprüche, **dadurch gekennzeichnet, dass** die Fraktion(en) solche Stoffklassen von Pflanzenextrakten enthalten ausgewählt aus der Gruppe Alkaloide, Bitterstoffe, Anthocyanine, Anthrachinone, Coumarine, Flavonoide, Glucosinolate, Lactone, Lignane, Lipide, Cannabinoide, Phenole, Polyphenole, Saponine, Terpene, Xanthone.

7. Verfahren zur Trennung und/oder Reinigung von Naturstoffen aus Pflanzenextrakten nach einem der vorhergehenden Ansprüche, **dadurch gekennzeichnet, dass** mindestens ein Pflanzenextrakt ausgewählt ist aus der Gruppe der Gattungen *Equiseti, Juglandis, Millefolii, Quercus, Taraxaci, Althaeae, Matricariae, Centaurium, Levisticum, Rosmarinus, Angelica, Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis*, *Rumicis, Verbena, Sambucus, Gentiana, Cannabis, Silybum.*

8. Verfahren zur Trennung und/oder Reinigung von Naturstoffen aus Pflanzenextrakten nach Anspruch 7, **dadurch gekennzeichnet, dass** mindestens ein Pflanzenextrakt ausgewählt ist aus der Gruppe der Arten *Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamilleblüten)), *Centaurium erythraea (Tausendgüldenkraut), Levisticum officinale (Liebstöckel), Rosmarinus officinalis (Rosmarin), Angelica dahurica (Sibirische Engelwurz, PinYin Name: Baizhi), Angelica sinensis (Chinesische Angelika, PinYin Name: Danggui), Artemisia scoparia (Besen-Beifuß, PinYin Name: Yinchen), Astragalus membranaceus (var. Mongolicus) (Traganthwurzel, Chin.: Huang-Qi), Leonurus japonicus (Löwenohr, Chin.: T'uei), Salvia miltiorrhiza (Rotwurzel-Salbei, Chin.: Danshen), Saposhnikovia divaricata (Siler, PinYin Name: Fangfeng), Scutellaria baicalensis (Baikal-Helmkraut, Banzhilian), Siegesbeckia pubescens (Himmlisches Kraut, PinYin Name: Xixianciao), Armoracia rusticana (Meerettich), Capsicum sp.(Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfrüchte, Chin.: Chuan Lian Zi), Olea europaea (Olive), Pelargonium sp. (Pelagornien), Phytolacca americana (Kermesbeeren), Primula veris (Schlüsselblume), Salix sp. (Weide), Thymus L. (Thymian), Vitex agnus castus (Mönchspfeffer), Vitis vinifera (Edle Weinrebe),* Rumicis herba (Sorrel herb), *Verbena officinalis* (Eisenkraut), *Sambucus nigra* (Schwarzer Holunder), *Gentiana lutea* (Gelber Enzian), *Cannabis sativa* (Hanf), *Silybum marianum* (Mariendistel).

9. Verfahren zur Trennung und/oder Reinigung von Naturstoffen aus Pflanzenextrakten nach einem der vorhergehenden Ansprüche, **dadurch gekennzeichnet**, nach einem der vorhergehenden Ansprüche, **dadurch gekennzeichnet, dass** die stationäre Phase und mobile Phase aus mindestens zwei Lösungsmitteln ausgewählt sind aus der Gruppe Kohlenwasserstoffe mit 5 - 8 Kohlenstoffatomen, insbesondere n-Heptan, Acetonitril, Ethylacetat, t-Butylmethylether, Alkohole, insbesondere Methanol, Wasser, Ethanol, so dass ein zwei-Phasen-System erhalten werden kann.

10. Verfahren zur Trennung und/oder Reinigung von Naturstoffen aus Pflanzenextrakten nach Anspruch 9, **dadurch gekennzeichnet, dass** die Lösungsmittel sind:
a.) n-Heptan/Acetonitril
b.) n-Heptan/Ethylacetat/Acetonitril;
c.) n-Heptan/Ethylacetat/t-Butylmethylether/ Acetonitril;
d.) n-Heptan/Ethylacetat/Methanol/Wasser;
e.) n-Heptan/Ethanol/Wasser.
